# EUROPÄISCHE PATENTANMELDUNG

(11) **EP 3 756 765 A1**
(43) Veröffentlichungstag der Anmeldung: **30.12.2020**
(21) Anmeldenummer: 20182399.4
(22) Anmeldetag: 25.06.2020
(51) Int. Cl.: B01L 3/00, C12M 1/00, B65D 43/02

(54) **PROBENBEHÄLTER UND VERWENDUNG EINES PROBENBEHÄLTERS**

(30) Priorität: 27.06.2019 DE 102019117411
(71) Anmelder: Testo SE & Co. KGaA, 79853 Lenzkirch (DE)
(72) Erfinder: Strnad, Martin, 79263 Simonswald (DE); Schnur, Andreas, 78628 Rottweil (DE); Riemer, Joel, 79874 Breitnau (DE); Wiech, Oliver, 78166 Donaueschingen (DE)
(74) Vertreter: Mertzlufft-Paufler, Cornelius

(57) **Zusammenfassung**

Die Erfindung betrifft einen Probenbehälter (1), mit einem Probenaufnahmeraum (2) und einem Verschluss (3) zum dichten Verschließen des Probenaufnahmeraums (2), wobei der Probenbehälter (1) wenigstens zwei getrennt voneinander angeordnete Dichtungen (4, 5, 15, 16) aufweist, und/oder wobei der Probenaufnahmeraum (2) eine Unterteilung in wenigstens zwei, voneinander thermisch entkoppelte, durch Flüssigkeit verbindbare Kompartimente (6) aufweist, und/oder wobei der Probenbehälter (1) an einer Außenwandung (7) des Probenaufnahmeraums (2) wenigstens eine Kupplungsstelle (8) zum Andocken eines Zusatzbehälters (9) über eine passende Gegenkupplungsstelle (10) des Zusatzbehälters (9) aufweist, wobei die Außenwandung (7) wenigstens eine Sollbruchstelle (11) aufweist, die bei einer Kopplung mit dem Zusatzbehälter (9) durchstoßen wird.

## Beschreibung

Die Erfindung betrifft einen Probenbehälter, mit einem Probenaufnahmeraum und einem Verschluss zum Verschließen des Probenaufnahmeraums, wobei der Probenbehälter beispielsweise zur Kultivierung von Mikroorganismen und/oder zur Durchführung einer chemischen Reaktion geeignet sein kann. Insbesondere kann der Probenbehälter zur Kultivierung und/oder zur Analyse, insbesondere zur chemischen Analyse, einer Probe geeignet sein.

Probenbehälter eingangs genannter Art sind bereits in einer Vielzahl von unterschiedlichen Ausführungsvarianten bekannt. So kennt man beispielsweise bereits Zellkulturschalen, Zellkulturflaschen und Mikrotiterplatten, die bei der Kultivierung tierischer oder pflanzlicher Zellen in einem Nährmedium verwendet werden. Üblicherweise werden die Probenaufnahmeräume dieser vorbekannten Probenbehälter jeweils einzeln mit einer zu kultivierenden und/oder analysierenden Probe bestückt, was bei einer großen Anzahl an unterschiedlichen Analysen zeitaufwändig sein kann. Um ideale Wachstumsbedingungen für Mikroorganismen oder ideale Reaktionsbedingungen zu erzeugen, werden die Probenbehälter häufig in Brutschränken (Inkubatoren) über einen gewissen Zeitraum inkubiert.

Nach Durchführung einer Analyse einer in einem Probenbehälter behandelten Probe ist es regelmäßig erforderlich, dass die Probe inaktiviert wird, da von den Proben häufig ein Gesundheitsrisiko ausgeht. In der Regel werden insbesondere biologische Proben durch eine thermische Behandlung im Überdruckbereich, beispielsweise in einem Autoklav, abgetötet.

Zwar kennt man bereits eine große Anzahl an Probenbehältern. Allerdings weisen diese den Nachteil auf, dass sie entweder nur undicht verschließbar sind oder dass eine Entstehung eines höheren Innendrucks im Probenbehälter dazu führt, dass sich der Probenbehälter öffnet und Probenmaterial entweichen kann.

Ein weiterer Nachteil vorbekannter Probenbehälter besteht darin, dass es bei vorbekannten Probenbehältern mit mehreren Probenaufnahmeräumen, wie zum Beispiel bei Mikrotiterplatten, nicht möglich ist, die in den unterschiedlichen Probenaufnahmeräumen enthaltenen Proben durch unterschiedliche Temperaturen zu behandeln.

Darüber hinaus ist es bei der Durchführung einer Kultivierung und/oder Analyse von Probenmaterial mit vorbekannten Probenbehältern in der Regel erforderlich, dass diese Arbeiten durch ein speziell geschultes Personal ausgeführt werden. Durch die zu kultivierenden und/oder analysierenden Proben können Gesundheitsrisiken ausgehen. Treten daher aufgrund eines unsachgemäßen, unsauberen Umgangs mit den Proben Kontaminationen auf, können diese durch Personal verschleppt werden. Daher ist es in der Regel erforderlich, dass der Umgang mit solch kritischen Proben nur in abgeschlossenen Laboren durch geschulte Labormitarbeiter erfolgen darf. Aufgrund der recht komplexen Arbeitsschritte der gesamten Kultivierung, Analyse und Inaktivierung besteht ein hohes Risiko einer Kontamination der Umgebung und umgebenden Personen. Es ist daher wünschenswert, einen Probenbehälter zur Verfügung zu stellen, durch welchen das Risiko einer Kontamination für die Umgebung und umgebende Personen reduziert oder sogar ausgeräumt ist.

Es besteht somit die Aufgabe, einen Probenbehälter eingangs genannter Art bereitzustellen, bei welchem die zuvor genannten Nachteile wenigstens teilweise ausgeräumt sind und dessen Gebrauchseigenschaften gegenüber vorbekannten Probenbehälter daher verbessert sind.

Diese Aufgabe/n wird/werden durch einen Probenbehälter mit den Merkmalen nach Anspruch 1 und/oder Anspruch 2 und/oder Anspruch 3 gelöst.

Insbesondere wird erfindungsgemäß zur Lösung der Aufgabe ein Probenbehälter eingangs genannter Art vorgeschlagen, wobei mittels des Verschlusses der Probenaufnahmeraum luftdicht verschließbar ist und wobei der Probenbehälter wenigstens eine sich bei erhöhenden Innendruck selbstverstärkende Dichtung aufweist. Insbesondere kann durch den Verschluss ein druckdichtes Verschließen des Probenaufnahmeraums möglich sein. Somit kann erreicht werden, dass der Probenbehälter auch bei einer starken Erhitzung, beispielsweise in einem Autoklav bei 121 °C heißem, gesättigten Wasserdampf, den auf den Probenbehälter wirkenden Innendruck und/oder Außendruck Stand hält und verschlossen bleibt. Durch die sich bei erhöhenden Innendruck selbstverstärkende Dichtung kann dabei ein wichtiges Sicherheitskriterium erreicht werden, dass auch bei thermischer Behandlung einer im Probenbehälter enthaltenen Flüssigkeit sich diese nicht soweit ausdehnen kann, dass es zu einem Auslaufen und schließlich zu einer Kontamination der Umgebung kommt.

Insbesondere wird erfindungsgemäß weiter zur Lösung der Aufgabe ein Probenbehälter eingangs genannter Art vorgeschlagen, wobei der bereits zuvor genannte Probenaufnahmeraum eine Unterteilung in wenigstens zwei voneinander thermisch entkoppelte, durch Flüssigkeit verbindbare oder in Gebrauchsstellung verbundene Kompartimente aufweist. Dies hat den Vorteil, dass es somit möglich ist, eine unterschiedliche thermische Behandlung von in einem Probenbehälter (sequentielle Erwärmung), insbesondere von in einem Probenaufnahmeraum, bereitgehaltenem Probenmaterial zu ermöglichen. Bislang ist es erforderlich, dass Proben in separaten Probenbehältern vorgehalten werden, um diese thermisch unterschiedlich zu behandeln. Somit ist eine deutlich einfachere Handhabung der Proben und eine deutlich einfachere Durchführung einer Kultivierung und/oder Analyse einer Probe möglich.

Insbesondere wird erfindungsgemäß weiter zur Lösung der Aufgabe ein Probenbehälter eingangs genannter Art vorgeschlagen, wobei der Probenbehälter an einer Außenwandung des bereits zuvor genannten Probenaufnahmeraums wenigstens eine Kupplungsstelle zum einen Flüssigkeitstransfer ermöglichenden Andocken eines Zusatzbehälters über eine passende Gegenkupplungsstelle aufweist. Insbesondere kann die Außenwandung wenigstens eine Sollbruchstelle aufweisen, die dazu eingerichtet ist, bei einer Kopplung mit dem Zusatzbehälter durchstoßen zu werden. Dies hat den Vorteil, dass somit auf einfache Weise eine Verbindung, insbesondere eine Flüssigkeitsverbindung, zwischen dem Probenaufnahmeraum und dem Zusatzbehälter über eine an der Sollbruchstelle entstandene Durchbruchöffnung hergestellt ist. Besonders vorteilhaft kann es sein, wenn die Verbindung zwischen dem Probenaufnahmeraum und dem Zusatzbehälter luftdicht, insbesondere druckdicht, ausbildbar oder in Gebrauchsstellung ausgebildet ist. Durch die Kopplung von Zusatzbehälter und Probenbehälter ist zum Beispiel eine einfache Analyse von einer im Probenaufnahmeraum enthalten Probe möglich, insbesondere ohne dass der Probenaufnahmeraum während der Durchführung eines Analyseverfahrens geöffnet werden muss. Über die Durchbruchöffnung ist ein Zugang zum Probenbehälter geschaffen, so dass ein Kontakt zwischen einer Analysesubstanz aus dem Zusatzbehälter mit der Probe möglich ist. Somit ist die Durchführung der Analyse deutlich vereinfacht. Beispielsweise kann der Zusatzbehälter einen seitlichen Flusstest (engl. lateral flow test) insbesondere in Form eines Teststreifens und/oder eines Lateral-Flow Device (abgekürzt: LFD) aufweisen. Durch die Kopplung des Probenbehälters mit dem Zusatzbehälter kann Flüssigkeit aus dem Probenaufnahmeraum in den Zusatzbehälter strömen, so dass eine Analyse durchführbar ist. Vorzugsweise kann innerhalb des Probenbehälters vor der Einrichtung der Flüssigkeitsverbindung ein Überdruck herrschen und/oder innerhalb des Zusatzbehälters ein Unterdruck herrschen. Somit kann nach der Kopplung ein Einströmen von Flüssigkeit in den Zusatzbehälter erleichtert werden.

Alternativ oder ergänzend dazu kann der Zusatzbehälter auch eine Substanz enthalten, die über die zuvor beschriebene Flüssigkeitsverbindung in den Probenaufnahmeraum einführbar ist, indem der Zusatzbehälter mit dem Probenaufnahmeraum verbunden wird. Vorzugsweise kann innerhalb des Zusatzbehälters vor einer Kopplung ein Überdruck ausgebildet sein, der ein Einströmen von Flüssigkeit aus dem Zusatzbehälter in den Probenaufnahmeraum erleichtert.

Der Probenaufnahmeraum kann beispielsweise durch einen Becher ausgebildet sein. Der Becher kann hierbei insbesondere eine zylindrische Form aufweisen. Vorzugsweise kann der Probenaufnahmeraum aus einem Kunststoff, insbesondere einem unelastischen Kunststoff hergestellt sein.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 und/oder nach Anspruch 2 und/oder nach Anspruch 3 kombiniert werden können.

Gemäß einer vorteilhaften Weiterbildung kann der Verschluss als ein Schraubverschluss und/oder als ein Schnappverschluss mit wenigstens einem Schnapphaken ausgebildet sein. Somit ist ein einfaches, insbesondere mit einer Hand durchführbares Verschließen des Probenaufnahmeraums möglich.

Alternativ oder ergänzend kann gemäß einer weiteren vorteilhaften Ausgestaltung vorgesehen sein, dass der Verschluss einen Deckel mit einer Seitenwand aufweist, wobei ein Innenumfang der Seitenwand wenigstens teilweise an einem Außenumfang der Außenwandung anliegt und/oder dass der Verschluss einen Deckel mit einer eine oder die Außenwandung des Probenaufnahmeraums axial teilweise überragende Seitenwand aufweist. Somit ist ein noch besseres Verschließen des Probenaufnahmeraums möglich, vorzugsweise da der axial die Außenwandung des Probenaufnahmeraums überragende Seitenwandabschnitt des Deckels zur Aufnahme und/oder Ausbildung eines Dichtelements vorgesehen sein kann. Beispielsweise kann am Innenumfang der Seitenwand des Deckels und/oder am Außenumfang der Außenwandung wenigstens eine Dichtung ausgebildet und/oder angeordnet sein.

Gemäß einer weiteren vorteilhaften Ausgestaltung kann die wenigstens eine Dichtung als wenigstens eine Axialdichtung und/oder als wenigstens eine Radialdichtung ausgebildet sein. Somit ist ein besonders sicheres Abdichten des Probenaufnahmeraums möglich. Zum Beispiel kann die wenigstens eine Dichtung derart ausgebildet sein, dass der Probenbehälter bei Verschließen des Verschlusses durch eine erste Dichtung, insbesondere eine Axialdichtung und/oder eine Radialdichtung, luftdicht verschließbar ist, und/oder dass die sich bei erhöhenden Innendruck selbstverstärkende Dichtung als zweite Dichtung, insbesondere als Axialdichtung und/oder als Radialdichtung, ausgestaltet ist.

Um ein Öffnen des Probenbehälters nach bereits ordnungsgemäß erfolgtem Verschließen des Probenbehälters verhindern zu können, kann der Verschluss des Probenbehälters als ein Sicherheitsverschluss ausgestaltet sein, mittels welchem der Probenaufnahmeraum irreversibel verschließbar ist, insbesondere so dass der Probenaufnahmeraum nach dem Verschließen bei sachgemäßem Gebrauch nicht mehr öffenbar ist.

Damit ein Anwender erkennen kann, ob der Probenbehälter ordnungsgemäß verschlossen ist, so dass es zu keiner Kontamination durch eine aus dem nicht ordnungsgemäß verschlossenen Probenaufnahmeraum auslaufenden Substanzen kommt, kann der Probenbehälter ein Sicherungsmittel aufweisen, durch welches nach dem Verschließen eine haptische und/oder visuelle Rückmeldung für einen Anwender gegeben ist, so dass der Anwender ein ordnungsgemäßes Verschließen erkennt. Der Anwender erkennt somit gleich, wenn der Probenaufnahmeraum mittels des Verschlusses nicht vollständig geschlossen ist. Der Anwender wird daher erst dann eine weitere Bearbeitung von einer im Probenbehälter enthaltenen Probe vornehmen, wenn durch das Sicherungsmittel angezeigt ist, dass der Probenaufnahmeraum ordnungsgemäß verschlossen ist. Ferner kann der Anwender durch das Sicherungsmittel feststellen, ob es für ihn und weitere Personen in seiner Umgebung sicher ist, mit dem Probenbehälter eine Laborumgebung zu verlassen, beispielsweise auch um eine weitere Bearbeitung insbesondere durch ungeschultes Personal außerhalb des Labors zu erleichtern. Durch das Sicherungsmittel kann zudem zusätzlich gewährleistet werden, dass es zu keiner nachträglichen Kontamination der Probe im Probenaufnahmeraum kommt, was ein Analyseergebnis verfälschen würde.

Um eine Probenverteilung innerhalb des Probenaufnahmeraums zu erleichtern, können die in Gebrauchsstellung durch Flüssigkeit verbundenen Kompartimente durch von einem Boden des Probenaufnahmeraum abstehende Kompartimentwände ausgebildet sein. Vorzugsweise können die Kompartimentwände gemessen vom Boden eine geringere Höhe als wenigstens eine den Probenaufnahmeraum begrenzende Wandung haben. Somit ist es möglich, dass eine in ein Kompartiment des Probenaufnahmeraums eingebrachte Probe ausreicht, um das Probenmaterial der Probe anschließend in wenigstens ein weiteres Kompartiment zu verteilen. Die Handhabung der Probe ist dadurch deutlich vereinfacht, da nach Verschließen des Probenaufnahmeraums ein einfaches Bewegen, wie z.B. Schütteln und/oder Schwenken, ausreicht, um das Probenmaterial der Probe möglichst gleichmäßig in den Kompartimenten zu verteilen.

Um sicherzustellen, dass die Kompartimente vollständig oder nahezu vollständig voneinander thermisch entkoppelt sind, können die Kompartimentwände beabstandet zueinander angeordnet sein und/oder sich gegenseitig nicht kontaktieren.

Um beispielsweise mittels eines Zusatzbehälters eine Analyse einer in einem Kompartiment enthaltenen Probe vornehmen zu können, kann es vorteilhaften sein, wenn wenigstens ein Kompartiment, insbesondere jedes Kompartiment, eine zugeordnete Kupplungsstelle aufweist. Alternativ oder ergänzend dazu kann wenigstens ein Kompartiment, insbesondere jedes Kompartiment, eine Sollbruchstelle aufweisen. Dies hat den Vorteil, dass die in unterschiedlichen Kompartimenten enthaltenen Proben unabhängig voneinander getestet werden können.

Gemäß einer weiteren vorteilhaften Ausgestaltung kann es dabei vorgesehen sein, dass die wenigstens eine Sollbruchstelle in radialer und/oder in axialer Richtung orientiert ist und/oder dass die wenigstens eine Sollbruchstelle bei einer Kopplung des Probenbehälters mit einem Zusatzbehälter in radialer und/oder in axialer Richtung durchbrochen wird. Eine Orientierung in eine bestimmte Richtung kann in diesem Zusammenhang bedeuten, dass ein auf einer Fläche stehender Normalenvektor in diese Richtung zeigt.

Gemäß einer besonders vorteilhaften Ausgestaltung kann die wenigstens eine sich bei erhöhenden Innendruck selbstverstärkende Dichtung durch einen sich in Richtung einer Behälteröffnung verjüngenden Außenwandungsbereich des Probenaufnahmeraums ausgebildet sein, insbesondere wobei eine Wandstärke des Außenwandungsbereich in Richtung der Behälteröffnung abnimmt, insbesondere kontinuierlich abnimmt. Beispielsweise kann sich der Außenwandungsbereich in Schließstellung verformen, vorzugsweise wenn ein Innendruck-Schwellenwert erreicht ist und/oder wenn eine Verdrängung des Außenwandungsbereichs durch Verschließen des Probenaufnahmeraums mit dem Deckel erfolgt.

Alternativ oder ergänzend dazu kann gemäß einer weiteren vorteilhaften Ausgestaltung vorgesehen sein, dass die wenigstens eine sich bei erhöhenden Innendruck selbstverstärkende Dichtung durch eine an einem Außenumfang einer Außenwandung, beispielsweise der bereits zuvor genannten Außenwandung, des Probenaufnahmeraums ausgebildete, insbesondere umlaufende, Materialausnehmung und einer die Außenwandung des Probenaufnahmeraums, insbesondere axial teilweise überragende, Seitenwand eines Deckels, beispielsweise des bereits vorgenannten Deckels, ausgebildet ist. Vorzugsweise wobei ein Innenumfang der Seitenwand über wenigstens einen Kontaktpunkt, insbesondere einen Steg, am Außenumfang der Außenwandung abgestützt ist. Der wenigstens eine Kontaktpunkt kann zusätzlich als Dichtelement, insbesondere als radial und/oder axial wirkendes Dichtmittel, wirken oder ausgebildet sein. Beispielsweise kann der wenigstens eine Kontaktpunkt am Außenumfang der Außenwandung und/oder am Innenumfang der Seitenwand des Deckels ausgebildet sein.

Gemäß einer weiteren vorteilhaften Ausgestaltung, kann die wenigstens eine sich bei erhöhenden Innendruck selbstverstärkende Dichtung durch einen Ringschnappverbinder ausgebildet sein. Somit ist eine besonders sichere Abdichtung auch bei besonders hohen Drücken möglich.

Die Erfindung betrifft zudem eine Verwendung eines Probenbehälters, wie er hierin beschrieben und beansprucht ist, zur Kultivierung und/oder zur Analyse, insbesondere zur chemischen Analyse, einer Probe, wobei eine Flüssigkeit, insbesondere ein Nährmedium, im Probenaufnahmeraum eingefüllt wird und dass eine Probe in wenigstens einem Kompartiment eines Probenaufnahmeraums angeordnet wird. Wie bereits zuvor in Bezug auf den Probenbehälter beschrieben wurde, ist somit eine Verteilung des Probenmaterials in den Kompartimenten durch einfache Bewegungen des gesamten Probenbehälters, wie beispielsweise Schwenken und/oder Schütteln, möglich. Der Probenbehälter kann dabei verschlossen bleiben. Zudem muss nicht jedes Kompartiment einzeln mit Probenmaterial bestückt werden. Somit ist die Handhabung deutlich vereinfacht.

Gemäß einer vorteilhaften Weiterbildung kann vorgesehen sein, dass eine Erwärmung, insbesondere eine sequenzielle Erwärmung, der Flüssigkeit und/oder der Probe in einem der Kompartimente vorgenommen wird. Insbesondere kann dabei eine Temperatur der Flüssigkeit und/oder der Proben in wenigstens einem anderen Kompartiment, insbesondere in allen weiteren Kompartimenten nahezu unverändert oder unverändert bleiben. Eine sequenzielle Erwärmung kann bedeuten, dass die in unterschiedlichen Kompartimenten enthaltenen Proben thermisch unterschiedlich behandelbar sind. Beispielsweise kann die thermische Behandlung durch Infrarotstrahlung vorzugsweise mit einer Infrarotheizung vorgenommen werden. Dies hat den Vorteil, dass mit den in unterschiedlichen Kompartimenten enthaltenen Proben unterschiedliche Analyseverfahren durchführbar sind.

Wie bereits zuvor erläutert, kann gemäß einer vorteilhaften Weiterbildung vorgesehen sein, dass durch die Flüssigkeit, insbesondere die die Kompartimentwände überflutende Flüssigkeit, eine Verteilung, insbesondere eine gleichmäßige Verteilung, der Probe von einem Kompartiment in wenigstens ein weiteres Kompartiment vorgenommen wird, insbesondere indem der Probenbehälter geschüttelt und/oder geschwenkt wird.

Um die besonders gute Verteilung von Probenmaterial, das in ein Kompartiment oder einen Zwischenraum zwischen den Kompartimenten eingebracht wird, in den übrigen Kompartimenten zu ermöglichen, kann eine vorteilhafte Ausgestaltung vorsehen, dass eine Flüssigkeit, insbesondere ein Nährmedium, in den Probenaufnahmeraum eingefüllt wird, so dass ein oberer Rand der Kompartimentwände der Kompartimente jeweils unterhalb eines Flüssigkeitspegels liegt.

Die Erfindung betrifft also insbesondere einen Probenbehälter, mit einem Probenaufnahmeraum und einem Verschluss zum dichten Verschließen des Probenaufnahmeraums, wobei der Probenbehälter wenigstens zwei getrennt voneinander angeordnete Dichtungen aufweist, und/oder wobei der Probenaufnahmeraum eine Unterteilung in wenigstens zwei voneinander thermisch entkoppelte, durch Flüssigkeit verbindbare Kompartimente aufweist, und/oder wobei der Probenbehälter an einer Außenwandung des Probenaufnahmeraums wenigstens eine Kupplungsstelle zum Andocken eines Zusatzbehälters über eine passende Gegenkupplungsstelle des Zusatzbehälters aufweist, wobei die Außenwandung wenigstens eine Sollbruchstelle aufweist, die bei einer Kopplung mit dem Zusatzbehälter durchstoßen wird.

Die Erfindung wird nun anhand mehrerer Ausführungsbeispiele näher beschrieben, ist jedoch nicht auf diese Ausführungsbeispiele beschränkt. Weitere Ausführungsbeispiele ergeben sich durch die Kombination der Merkmale einzelner oder mehrerer Ansprüche untereinander und/oder mit einzelnen oder mehreren Merkmalen der Ausführungsbeispiele.

Es zeigt:

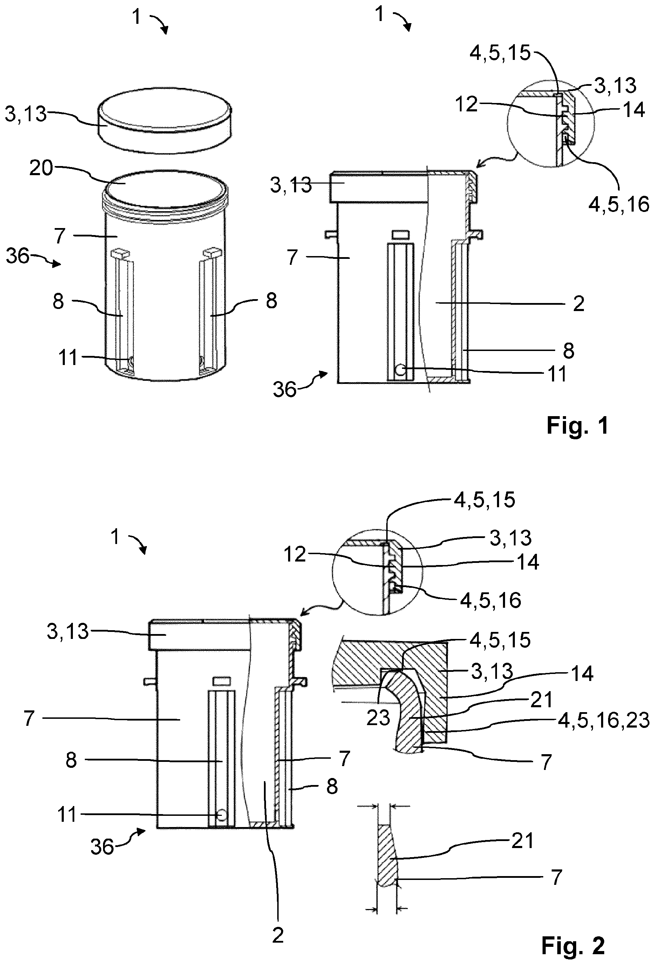

- Fig. 1: eine erste Ausgestaltungsvariante eines erfindungsgemäßen Probenbehälters, mit einer ersten Dichtung und einer zweiten Dichtung in perspektivischer Darstellung und in einer teils geschnittenen Seitenansicht, wobei zwischen einem als Deckel ausgebildeten Verschluss und einem Außenwandbereich mittels wenigstens eines Schnapphakens eine irreversible Rastverbindung eingerichtet ist, so dass der Verschluss nach einem ordnungsgemäßen Verschließen nicht mehr öffenbar ist;
- Fig. 2: eine zweite Ausführungsvariante eines erfindungsgemäßen Probenbehälters, wobei eine Wandstärke eines Außenwandbereichs in Richtung einer Behälteröffnung abnimmt und sich daher im Längsschnitt verjüngt, wobei der Außenwandbereich wenigstens teilweise radial nach innen verformt ist, so dass ein Randbereich der Behälteröffnung zusammen mit dem Deckel eine axial wirkende Dichtung (Axialdichtung) und ein Seitenwandbereich des Deckels über seinen Innenumfang eine radial wirkende Dichtung (Radialdichtung) mit einem Außenumfang einer Außenwandung des Probenaufnahmeraums ausbildet;

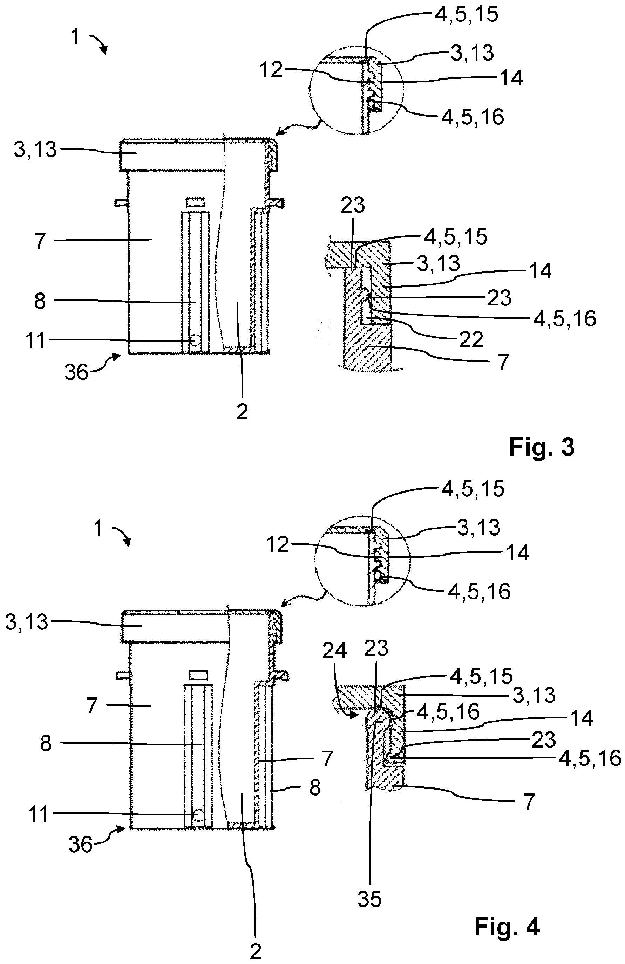

- Fig. 3: eine dritte Ausführungsvariante eines erfindungsgemäßen Probenbehälters, wobei eine Wandstärke eines Außenwandbereichs in Richtung einer Behälteröffnung abnimmt und sich daher im Längsschnitt verjüngt, wobei ein Randbereich der Behälteröffnung zusammen mit dem Deckel eine axial wirkende Dichtung (Axialdichtung) und ein Seitenwandbereich des Deckels über seinen Innenumfang eine radial wirkende Dichtung (Radialdichtung) mit einem Außenumfang einer Außenwandung des Probenaufnahmeraums ausbildet, wobei der Außenwandbereich und/oder der Deckel wenigstens ein Dichtmittel aufweist/aufweisen, über welches wenigstens ein Kontaktpunkt mit der Seitenwand des Deckels in Schließstellung hergestellt ist;
- Fig. 4: eine vierte Ausführungsvariante eines erfindungsgemäßen Probenbehälters, wobei eine Wandstärke eines Außenwandbereichs in Richtung einer Behälteröffnung zunächst abnimmt und dann an einem Wulst erneut zunimmt, wobei ein Randbereich der Behälteröffnung zusammen mit dem Deckel eine axial wirkende Dichtung (Axialdichtung) und ein Seitenwandbereich des Deckels über seinen Innenumfang eine radial wirkende Dichtung (Radialdichtung) mit einem Außenumfang einer Außenwandung des Probenaufnahmeraums ausbildet, wobei der Außenwandbereich und/oder der Deckel wenigstens ein Dichtmittel aufweist/aufweisen, über welches wenigstens ein Kontaktpunkt mit der Seitenwand des Deckels in Schließstellung hergestellt ist;

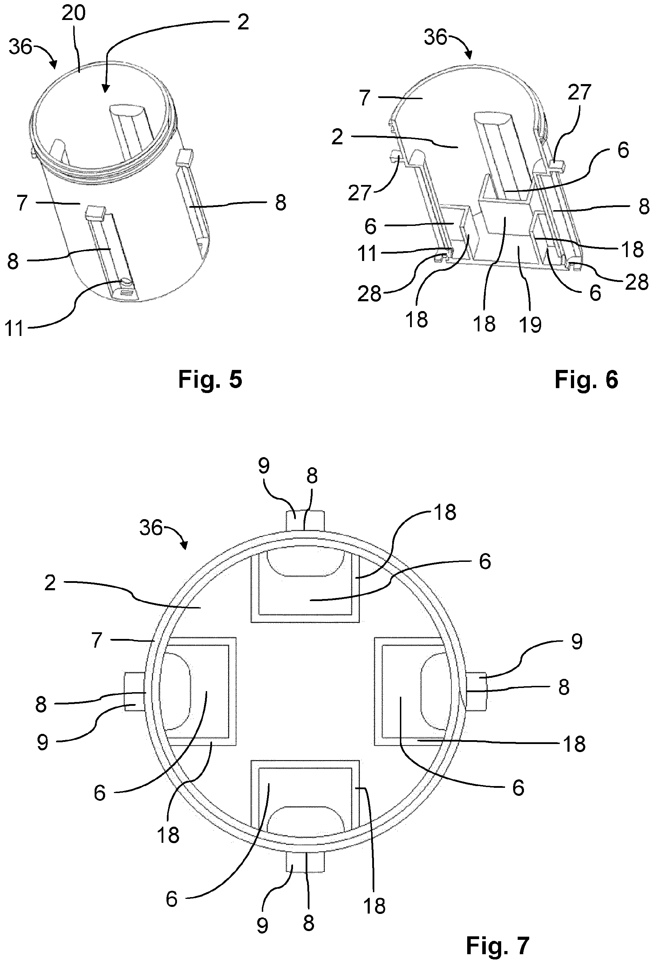

- Fig. 5: eine perspektivische Darstellung einer möglichen Ausgestaltungsform eines Probenbehälters ohne Verschluss;
- Fig. 6: eine perspektivische Darstellung eines Längsschnitts der Ausgestaltungsform des Probenbehälters aus Fig. 5, wobei der Probenaufnahmeraum in vier voneinander thermisch entkoppelte Kompartimente unterteilt ist;
- Fig. 7: eine Draufsicht auf den Probenbehälter aus den Figuren 5 und 6;

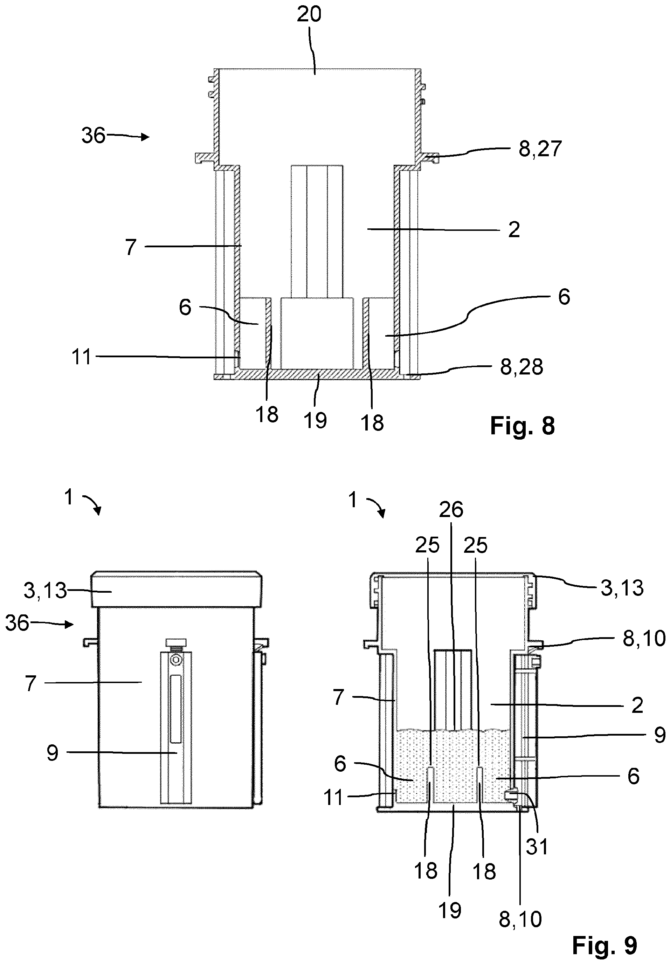

- Fig. 8: eine Seitenansicht eines Längsschnitts der Ausgestaltungsform des Probenbehälters aus den Figuren 5-7, wobei der Probenaufnahmeraum in vier voneinander thermisch entkoppelte Kompartimente unterteilt ist;
- Fig. 9: eine fünfte Ausführungsvariante eines Probenbehälters, der zur sequenziellen Erhitzung seiner Kompartimente eingerichtet ist, indem diese räumlich getrennt voneinander innerhalb des Probenaufnahmeraums angeordnet sind, wobei ein Flüssigkeitspegel während des Gebrauchs des Probenbehälters innerhalb des Probenaufnahmeraums höher als ein oberer Rand der Kompartimentwände steht;

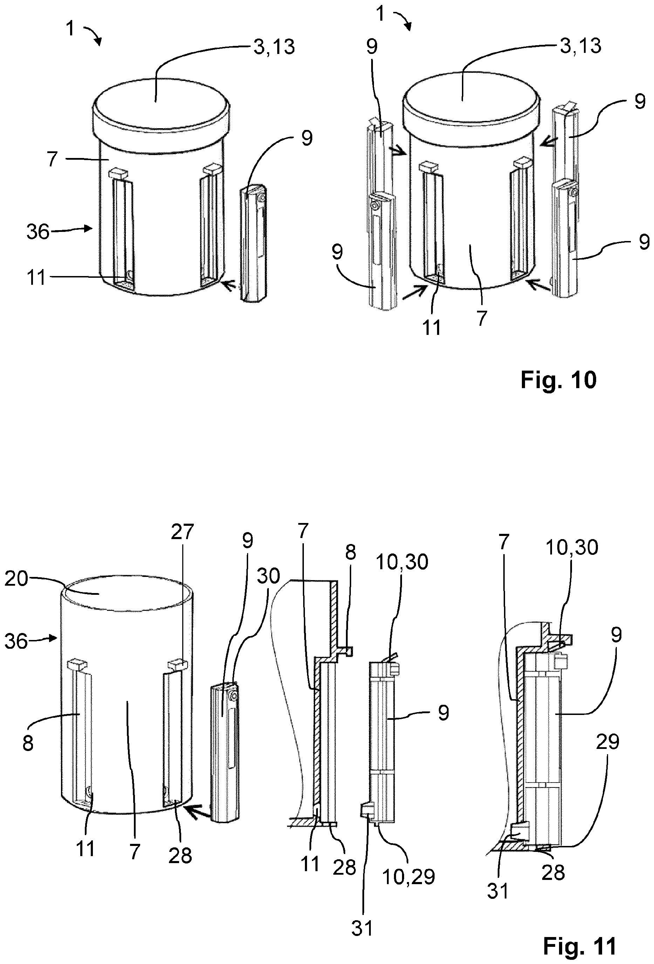

- Fig. 10: eine sechste Ausführungsvariante eines Probenbehälters, wobei der Probenbehälter eine durch einen Rasthaken, eine Einrastausnehmung und/oder eine Einbuchtung in der Außenwandung ausgebildete Kupplungsstelle aufweist, die dazu eingerichtet ist, um mit einer dazu passenden Gegenkupplungsstelle eines Zusatzbehälters verbunden zu werden, um den Probenbehälter mit dem Zusatzbehälter insbesondere irreversibel zu verbinden;
- Fig. 11: die Ausführungsvariante aus Fig. 10, wobei die Außenwandung des Probenbehälters eine Sollbruchstelle aufweist, die bei einer Kopplung des Probenbehälters mit dem Zusatzbehälter mittels eines Durchbruchelements durchbrochen wird, so dass eine Flüssigkeitsverbindung zwischen dem Probenaufnahmeraum und dem Zusatzbehälter entsteht;

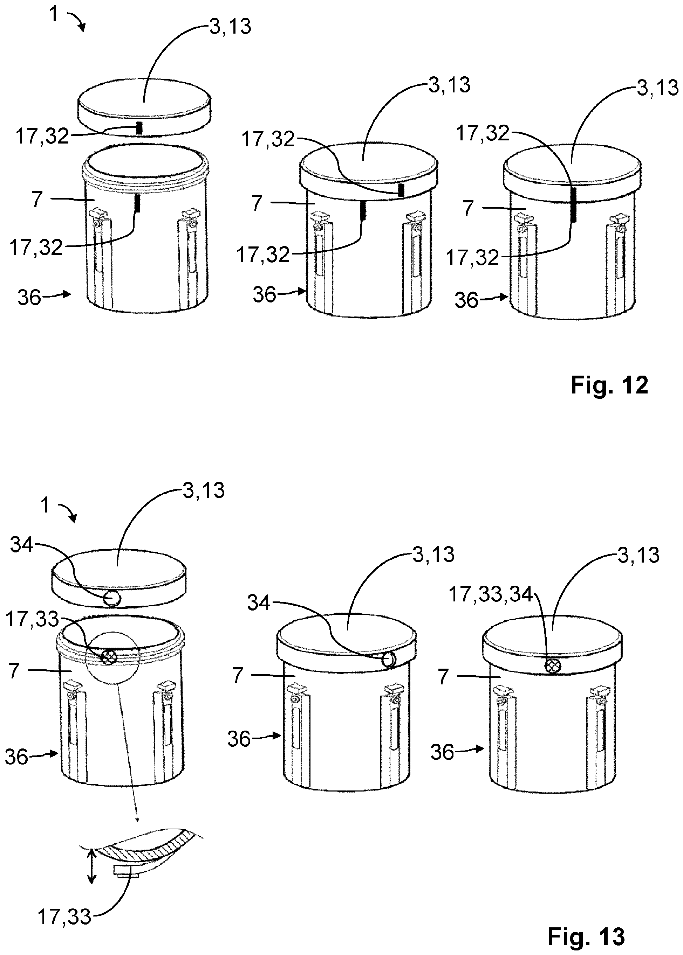

- Fig. 12: eine siebte Ausführungsvariante eines Probenbehälters, wobei am Verschluss und an der Außenwandung des Probenaufnahmeraums jeweils ein als Markierungen ausgebildetes Sicherungsmittel vorliegt, wobei die beiden Markierungen bei einem ordnungsgemäß verschlossenen Probenaufnahmeraum zueinander fluchtend angeordnet sind;
- Fig. 13: eine achte Ausführungsvariante eines Probenbehälters, wobei der Verschluss ein Loch aufweist, welches nur bei einem ordnungsgemäß verschlossenen Probenaufnahmeraum den Blick auf ein sichtbarwerdendes Verschlusselement an der Außenwandung freigibt.

In den Figuren 1 bis 13 sind mehrere mögliche Ausführungsvarianten eines erfindungsgemäßen, im Ganzen als 1 bezeichneten Probenbehälters gezeigt.

Der Probenbehälter 1 ist in den gezeigten Ausführungsvarianten wenigstens zweiteilig ausgebildet und weist einen Probenaufnahmeraum 2 und einen als Deckel 13 ausgebildeten Verschluss 3 zum Verschließen des Probenaufnahmeraum 2 auf. Es ist jedoch auch möglich, dass der Verschluss 3 untrennbar mit dem Probenaufnahmeraum 2 verbunden ist. Der Deckel 13 weist eine umlaufende Seitenwand 14 auf, die in Schließstellung eine Außenwandung 7 des Probenaufnahmeraums 2 teilweise, insbesondere in axialer Richtung, überlappt.

Der Probenaufnahmeraum 2 kann beispielsweise als ein Becher 36 ausgebildet sein. Der Becher 36 kann hierbei insbesondere eine zylindrische Form aufweisen. Insbesondere kann der Becher 36 steif sein.

Zum Verschließen des Probenaufnahmeraum 2 weist der Probenbehälter 1 beispielsweise einen Schraubverschluss auf, der ein Innengewinde hat, das zu einem Außengewinde an einer Außenwandung 9 des Probenaufnahmeraums 2 passt.

Alternativ oder ergänzend dazu kann der Probenbehälter 1 einen Schnappverschluss, also insbesondere einen Schraubschnappverschluss, aufweisen. Hierbei ist es möglich, den Schnappverschluss derart auszubilden, dass der Probenaufnahmeraum 2 mittels des Verschlusses 3 irreversibel, also nicht öffenbar verschließbar ist. Der Schnappverschluss kann hierbei beispielsweise an dem Deckel 13 einen oder mehrere Schnapphaken 12 aufweisen, der/die in eine Ausnehmung oder mehrere Ausnehmungen in der Außenwandung 7 eingreift/eingreifen und/oder einen Vorsprung an der Außenwandung 7 hintergreift/hintergreifen. Es ist jedoch auch denkbar, dass das beschriebene Prinzip umgekehrt wird, so dass der oder die Schnapphaken 12 alternativ oder ergänzend an der Außenwandung 7 ausgebildet ist/sind.

In den Figuren 1-4 sind mehrere Ausführungsvarianten eines Probenbehälters 1 dargestellt, die wenigstens eine sich bei erhöhenden Innendruck selbstverstärkende Dichtung 4, 5, 15, 16 aufweisen. Durch ein Zusammenwirken des Verschlusses 3 mit der Außenwandung 7 des Probenaufnahmeraums 2 werden diese bei erhöhtem Innendruck zumindest in einem Kontaktpunkt 23 und/oder über ein Dichtmittel 23 in axialer und/oder radialer Richtung stärker aneinandergepresst. Der Kontaktpunkt 23 kann beispielsweise eine Kontaktfläche und/oder eine Kontaktlinie sein, vorzugsweise eine in Umfangsrichtung umlaufende Kontaktfläche.

Die Dichtungen 4, 5, 15, 16 können jeweils durch angeformtes Material an der Außenwandung 7 und/oder am Verschluss 3 und/oder durch zwischen dem Verschluss 3 und der Außenwandung 7 angeordnete, verformbare Dichtmittel 23, wie beispielsweise Dichtringe, ausgebildet sein.

Bei den in den Figuren 1-4 dargestellten Ausführungsvarianten des Probenbehälters 1 sind wenigstens eine erste Dichtung 4 und eine zweite Dichtung 5 vorgesehen. Somit weist der Probenbehälter 1 eine zweistufige Dichtung auf. Durch die erste Dichtung 4 kann eine bereits flüssigkeitsdichte Abdichtung des Probenaufnahmeraums 2 durch Verschließen mittels des Deckels 13 eingerichtet sein. Durch die zweite Dichtung 5 ist es schließlich möglich, eine noch bessere Abdichtung des Probenaufnahmeraums 2 einzurichten, die insbesondere druckdicht ist. Somit ist es möglich, einen mit Flüssigkeit gefüllten Probenbehälter 1 hohen Temperaturen, wie beispielsweise bei einem Autoklaviervorgang, auszusetzen, ohne dass dieser undicht wird.

Die erste Dichtung 4 ist in den gezeigten Beispielen als eine in radialer Richtung wirkende Dichtung (Radialdichtung 16) ausgebildet. Dabei kann dies beispielsweise durch einen Dichtring und/oder durch an der Außenwandung 7 des Probenaufnahmeraums 2 angeformte Dichtmittel 23 ausgebildet sein. Die erste Dichtung 4 kann jedoch auch als eine in axialer Richtung wirkende Dichtung (Axialdichtung 15) ausgebildet sein.

Die zweite Dichtung 5 ist in den gezeigten Beispielen als eine in axialer Richtung wirkende Dichtung (Axialdichtung 15) ausgebildet. Die Axialdichtung 15 kann dabei durch ein auf einem Rand der Behälteröffnung 20 angeordnetes und/oder angeformtes Dichtelement, wie beispielsweise einem Dichtring, ausgebildet sein. Die zweite Dichtung 5 kann jedoch auch als eine in radialer Richtung wirkende Dichtung (Radialdichtung 16) ausgebildet sein.

Figur 2 zeigt eine Ausführungsvariante des Probenbehälters 1, dessen Außenwandung 7 eine in Richtung der Behälteröffnung 20 abnehmende Wandstärke aufweist, somit also einen sich im Längsschnitt verjüngenden, insbesondere kontinuierlich verjüngenden, Außenwandbereich 21 aufweist. Durch die Verringerung der Wandstärke kann eine Sollbiegestelle ausgebildet werden. Die beiden Dichtungen 4, 5, 15, 16 sind dabei durch eine Verformung des Außenwandbereichs 21 des Probenaufnahmeraums 2 in radialer Richtung, vorzugweise durch ein nach innen Biegen des Außenwandbereichs 21, ausgebildet. Die Verformung des Außenwandbereichs 21 kann alternativ oder ergänzend zumindest teilweise durch einen auf den Deckel 13 und den Außenwandbereich 21 im Inneren des Probenaufnahmeraums 2 wirkenden Innendruck bewirkt werden. Alternativ oder ergänzend kann es zu einer anfänglichen Verformung kommen, wenn der Probenaufnahmeraum 2 mit dem Deckel 13 verschlossen wird, insbesondere zugeschraubt wird (auch wenn im Inneren kein erhöhter Druck herrscht).

Durch die Verformung können wenigstens zwei Kontaktpunkte/Dichtmittel 23 zwischen dem Deckel 13 und der Außenwandung 7 ausgebildet sein. Ein durch die Außenwandung 7 ausgebildeter Randbereich der Behälteröffnung 20 ist dabei so verformt, dass er den Deckel 13 nur mit einer Kante beaufschlagt und dabei ein Dichtmittel 23 und/oder einen Kontaktpunkt 23 ausbildet. Somit ist hier eine Axialdichtung 15 ausgestaltet.

Ein weiterer Kontaktpunkt und/oder ein weiteres Dichtmittel 23 ist zwischen dem Außenumfang der Außenwandung 7 und dem Innenumfang einer Seitenwand 14 des Deckels 13 ausgebildet. Dadurch ist eine Radialdichtung 16 ausgestaltet.

Steigt der Innendruck, so werden der Deckel 13 und die Außenwandung 7 zumindest in den wenigstens zwei Kontaktpunkten 23 stärker gegeneinandergepresst. Dieses Wirkprinzip ist auch bei den Ausführungsvarianten aus den Figuren 3 und 4 umgesetzt, wobei sich die jeweilige strukturelle Ausgestaltung unterscheidet. Es ist jedoch möglich, die Merkmale der Ausführungsvarianten der Figuren 2 bis 4 miteinander zu einer neuen Ausführungsvariante zu kombinieren.

Figur 3 zeigt eine Ausführungsvariante des Probenbehälters 1, dessen Außenwandung 7 eine in Richtung der Behälteröffnung 20 im Bereich einer Materialausnehmung 22 abnehmende Wandstärke, insbesondere mit einer Stufe, aufweist. Die Materialausnehmung 22 kann beispielsweise als in Umlaufrichtung umlaufende Nut ausgebildet sein. Am Außenumfang der Außenwandung 7 ist ein vorzugsweise stegförmiges und/oder umlaufendes Dichtmittel 23 ausgebildet, über welches ein Kontaktpunkt 23 mit der Seitenwand 14 des Deckels 13 in Schließstellung eingerichtet ist. Somit ist eine Radialdichtung 16 ausgebildet.

Der Randbereich der Behälteröffnung 20 bildet zusammen mit dem Deckel 13 wenigstens einen weiteren Kontaktpunkt 23 aus. Ein am wenigstens einen weiteren Kontaktpunkt 23 ausbildendes Dichtmittel kann am Deckel 13 und/oder am Rand der Behälteröffnung 20 ausgebildet, insbesondere angeformt, und/oder angeordnet sein. Somit ist eine Axialdichtung 15 ausgebildet.

Erhöht sich der Innendruck im Probenaufnahmeraum 2, kommt es auch hier zu einer Verformung der Außenwandung 7, wodurch sich die Dichtungen 4, 5, 15 16 selbst verstärken, indem sich der Anpressdruck zwischen der Außenwandung 7 und dem Deckel 13 und/oder zwischen dem Rand, insbesondere einer Stirnseite, der Behälteröffnung 20 und dem Deckel 13 zumindest in den Kontaktpunkten 23 verstärkt.

Figur 4 zeigt eine weitere Ausführungsvariante eines Probenbehälters 1, der einen Verschluss 3 mit einem Ringschnappverbinder 24 aufweist. Am Rand der Behälteröffnung 20 ist ein als Wulst 35 ausgestaltetes Dichtmittel 23 ausgebildet, das in Schließstellung des Probenbehälters 1 wenigstens einen Kontaktpunkt 23, vorzugsweise wenigstens einen axial wirkenden und/oder einen radial Wirkenden Kotaktpunkt 23, mit dem Deckel 13 ausbildet.

Der Deckel 13, insbesondere der zuvor beschriebene Ringschnappverbinder 24, weist am Randbereich der Seitenwand 14 des Deckels 13 einen radial nach innen gerichteten Vorsprung auf, der in Schließstellung in eine dazu korrespondierende Ausnehmung in der Außenwandung 7 eingreift und eine, vorzugsweise irreversible, Rastverbindung ausbildet. Somit ist hier eine (weitere) Radialdichtung 16 ausgebildet. Gleichzeitig können der Deckel 13 und der Becher 36 durch die entstehende Rastverbindung miteinander untrennbar verbunden werden. Durch die in Schließstellung des Probenbehälters 1 ausgebildete Rastverbindung ist also bei ordnungsgemäßem Gebrauch das Öffnen des Probenaufnahmeraums 2 nicht mehr möglich.

Der in den Figuren 5-9 gezeigte Probenbehälter 1 weist einen in vier Kompartimente 6 untergliederten Probenaufnahmeraum 2 auf, wobei die Kompartimente 6 durch einen Zwischenraum (Freiraum) in der Mitte des Probenaufnahmeraums 2 voneinander thermisch isoliert sind.

Wie in Figur 9 gezeigt ist, ist es vorgesehen, dass bei ordnungsgemäßer Befüllung des Probenaufnahmeraum 2 mit einer Flüssigkeit, z.B. einem Nährmedium, ein Flüssigkeitspegel 26 gemessen von einem Boden 19 des Probenaufnahmeraums 2 oberhalb eines Randes 25 der Kompartimentwände 18 liegt. Die Kompartimentwände 18 bilden dabei zusammen mit der Außenwandung 7 die einzelnen Kompartimente 6 aus. Somit sind die Kompartimente 6 in Gebrauch über die Flüssigkeit miteinander verbunden.

Die den Probenaufnahmeraum 2 begrenzende Außenwandung 7 ist gemessen vom Boden 19 höher, als die einzelnen Kompartimentwände 18. Somit ist ein Überfluten der einzelnen Kompartimente 6 möglich. Dies hat den Vorteil, dass eine Probe lediglich in ein Kompartiment 6 eingeführt werden muss, der Probenbehälter 1 anschließend geschlossen werden kann und durch Schwenken und/oder Schütteln des Probenbehälters 1 eine nahezu gleichmäßige Verteilung des Probenmaterials in alle Kompartimente 6 möglich ist. Dies vereinfacht die Handhabung, insbesondere beim Umgang mit toxischen oder infektiösen Proben.

Aufgrund der thermischen Entkopplung der Kompartimente 6 ist es möglich, darin enthaltene Proben unabhängig voneinander zu erwärmen. Die Erwärmung kann hierbei beispielsweise durch eine Infrarotheizung erfolgen. Somit können unterschiedliche Kulturbedingungen und/oder Reaktionsbedingungen innerhalb eines Probenaufnahmeraums 2 geschaffen werden.

Jedes Kompartiment 6 weist eine innerhalb des Kompartiments 6 liegende und durch eine Außenwandung 7 ausgebildete Sollbruchstelle 11 auf. Die Sollbruchstellen 11 dienen dazu, um einen Flüssigkeitsaustausch zwischen einem mit dem Probenbehälter 1 koppelbaren Zusatzbehälter 9 auszubilden. In den Figuren 10 und 11 sind hierzu Ausführungsvarianten gezeigt. Probenbehälter 1 und Zusatzbehälter 9 können zusammen ein Set zur Kultivierung und Analyse einer Probe sein.

Der Probenbehälter 1 weist für jede Sollbruchstelle 11 eine Kupplungsstelle 8 auf. Wie in den Figuren 5, 6 und 7 gezeigt ist, kann die Kupplungsstelle 8 beispielsweise durch einen Rasthaken 27 und eine Einrastausnehmung 28 ausgebildet sein. Durch die Kupplungsstelle 8 kann somit eine Kopplung des Probenbehälters 1 über eine geeignete Gegenkupplungsstelle 10 mit dem Zusatzbehälter 9 eingerichtet werden. Die Kupplungsstelle 8 kann weiter eine an der Außenwandung 7 ausgebildete Aufnahmeausbuchtung für den Zusatzbehälter 9 aufweisen. Die Gegenkupplungsstelle 10 kann beispielsweise durch eine Rastnase 29, die in die Einrastausnehmung 28 in Schließstellung eingreift und ein Federelement 30, das durch den Rasthaken 27 in Schließstellung beaufschlagt wird, ausgebildet sein.

Der Zusatzbehälter 9 kann somit über die Rastnase 29 in die im Boden 19 ausgebildete Einrastausnehmung 28 eingesteckt werden. Durch die Rastnase 29 und die Einrastausnehmung 28 kann somit eine Drehachse ausgebildet werden. Der Zusatzbehälter 9 kann dann in radialer Richtung über ein Durchbruchelement 31 in die Sollbruchstelle 11 eingeführt werden. Dadurch wird die Sollbruchstelle 11 durch das Durchbruchelement 31 durchstoßen, so dass eine Flüssigkeitsverbindung zwischen dem Zusatzbehälter 9 und dem Probenaufnahmeraum 2 hergestellt ist. Die beschriebene Ausgestaltung hat den Vorteil, dass eine möglichst große Hebelwirkung erreicht werden kann, so dass das Durchbrechen der Sollbruchstelle 11 mit relativ geringem Kraftaufwand möglich ist, indem das obere Ende des Zusatzbehälters in einer Kreisbewegung in Richtung der Außenwandung 7 geführt wird, bis ein Verrasten des Rasthakens 27 mit dem Federelement 30 erfolgt.

Das Federelement 30 kann beispielsweise als ein schräg abstehender, vorgespannter Flügel ausgebildet sein, durch welchen eine Federkraft erzeugt wird, die beim Verrasten des Rasthakens 27 mit dem Federelement 30 überwunden werden muss.

Die Figuren 12 und 13 zeigen eine Ausführungsvariante eines Probenbehälters 1, der ein Sicherungsmittel 17 aufweist, durch welches nach dem Verschließen des Bechers 36 mit dem Deckel 13 durch einen Anwender wahrgenommen werden kann, ob der Probenaufnahmeraum 2 ordnungsgemäß, also insbesondere dicht, verschlossen ist. Durch das Sicherungsmittel 17 kann der Anwender taktil und/oder visuell wahrnehmen, ob der Probenbehälter 1 ordnungsgemäß verschlossen ist.

In Figur 12 ist eine Ausführungsvariante gezeigt, bei welcher auf dem Deckel 13, insbesondere auf der Außenseite der Seitenwand 14, eine Markierung in Form eines insbesondere senkrechten Striches aufgebracht ist. Der Becher 36 weist an einer Außenseite ebenfalls eine Markierung in Strichen auf. Wird der Becher 36 mit dem Deckel 13, beispielsweise durch eine Schraubverbindung und/oder eine Schnappverbindung, verschlossen, so wird der Deckel 13 relativ zum Becher 36 soweit gedreht, bis die beiden Markierungen übereinander liegen, also zueinander fluchtend angeordnet sind. Somit ist eine visuelle Ablesbarkeit eines ordnungsgemäß erfolgten Verschließens über das als Markierungen ausgebildete Sicherungsmittel 17 möglich.

In Figur 13 ist eine dazu ergänzende oder alternative Ausführungsvariante gezeigt. Der Deckel 13 weist in seiner Seitenwand 14 ein Loch 34 auf, das ähnlich dem Prinzip der zuvor beschriebenen Strichmarkierungen wirkt. Der Deckel 13 wird relativ zum Becher 36 soweit gedreht, bis das Loch 34 in Schließstellung über einer Markierung 32, beispielsweise einem sichtbar werdenden Verschlusselement 33, angeordnet ist. Der Anwender kann somit das Verschlusselement 33 nur bei ordnungsgemäßem Verschließen des Probenaufnahmeraums 2 sehen. Gleichzeitig kann das Verschlusselement 33 dazu eingerichtet sein, dass alternativ oder ergänzend zur visuellen Wahrnehmung eine haptische und/oder taktile Wahrnehmung durch Erfühlen des Verschlusselements 33 nur bei ordnungsgemäß verschlossenem Probenaufnahmeraum 2 möglich ist. Durch Verschließen des Probenaufnahmeraums 2 mit dem Verschluss 3 kann eine Verstellung des Verschlusselements 33 von einer ersten Stellung in eine zweite Stellung erfolgen. Dabei kann das Verschlusselement 33 derart eingerichtet sein, dass es in einer der beiden Stellungen durch einen Anwender erfühlt und/oder visuell wahrgenommen werden kann.

### Bezugszeichenliste

- 1: Probenbehälter
- 2: Probenaufnahmeraum
- 3: Verschluss
- 4: Erste Dichtung
- 5: Zweite Dichtung
- 6: Kompartiment
- 7: Außenwandung
- 8: Kupplungsstelle
- 9: Zusatzbehälter
- 10: Gegenkupplungsstelle
- 11: Sollbruchstelle
- 12: Schnapphaken
- 13: Deckel
- 14: Seitenwand
- 15: Axialdichtung
- 16: Radialdichtung
- 17: Sicherungsmittel
- 18: Kompartimentwand
- 19: Boden
- 20: Behälteröffnung
- 21: sich verjüngender Außenwandbereich
- 22: Materialausnehmung
- 23: Kontaktpunkt/Dichtmittel
- 24: Ringschnappverbinder
- 25: oberer Rand der Kompartimentwand
- 26: Flüssigkeitspegel
- 27: Rasthaken
- 28: Einrastausnehmung
- 29: Rastnase
- 30: Federelement
- 31: Durchbruchelement
- 32: Markierung
- 33: Sichtbarwerdendes Verschlusselement
- 34: Loch
- 35: Wulst
- 36: Becher

## Patentansprüche

1. Probenbehälter (1), mit einem Probenaufnahmeraum (2) und einem Verschluss (3) zum Verschließen des Probenaufnahmeraums (2), **dadurch gekennzeichnet, dass** mittels des Verschlusses (3) der Probenaufnahmeraum (2) luftdicht verschließbar ist und dass der Probenbehälter (1) wenigstens eine sich bei erhöhenden Innendruck selbstverstärkende Dichtung (4, 5, 15, 16) aufweist.

2. Probenbehälter (1) nach dem Oberbegriff aus Anspruch 1 oder nach Anspruch 1, **dadurch gekennzeichnet, dass** der Probenaufnahmeraum (2) eine Unterteilung in wenigstens zwei voneinander thermisch entkoppelte, durch Flüssigkeit verbindbare oder in Gebrauchsstellung durch Flüssigkeit verbundene Kompartimente (6) aufweist.

3. Probenbehälter (1) nach dem Oberbegriff aus Anspruch 1 oder nach einem der vorstehenden Ansprüche, **dadurch gekennzeichnet, dass** der Probenbehälter (1) an einer Außenwandung (7) des Probenaufnahmeraums (2) wenigstens eine Kupplungsstelle (8) zum einen Flüssigkeitstransfer ermöglichenden Andocken eines Zusatzbehälters (9) über eine passende Gegenkupplungsstelle (10) aufweist, insbesondere wobei die Außenwandung (7) wenigstens eine Sollbruchstelle (11) aufweist, die dazu eingerichtet ist, bei einer Kopplung mit dem Zusatzbehälter (9) durchstoßen zu werden.

4. Probenbehälter (1) nach einem der vorstehenden Ansprüche, **dadurch gekennzeichnet, dass** der Verschluss (3) als ein Schraubverschluss und/oder ein Schnappverschluss mit wenigstens einem Schnapphaken (12) ausgebildet ist und/oder dass der Verschluss (3) einen Deckel (13) mit einer Seitenwand (14) aufweist, wobei ein Innenumfang der Seitenwand (14) wenigstens teilweise an einem Außenumfang der Außenwandung (7) anliegt.

5. Probenbehälter (1) nach einem der vorstehenden Ansprüche, **dadurch gekennzeichnet, dass** die wenigstens eine Dichtung (4, 5, 15, 16) als wenigstens eine Axialdichtung (15) und/oder als wenigstens eine Radialdichtung (16) ausgebildet ist, insbesondere derart, dass der Probenbehälter (1) beim Verschließen des Verschlusses (3) durch eine erste Dichtung (4) luftdicht verschließbar ist, und/oder dass die sich bei erhöhendem Innendruck selbstverstärkende Dichtung (4, 15, 16) als eine zweite Dichtung (5) ausgestaltet ist.

6. Probenbehälter (1) nach einem der vorstehenden Ansprüche, **dadurch gekennzeichnet, dass** der Verschluss (3) des Probenbehälters (1) als Sicherheitsverschluss ausgestaltet ist, mittels welchem der Probenaufnahmeraum (2) irreversibel verschließbar ist, insbesondere so dass er nach dem Verschließen nicht mehr öffenbar ist.

7. Probenbehälter (1) nach einem der vorstehenden Ansprüche, **dadurch gekennzeichnet, dass** der Probenbehälter (1) ein Sicherungsmittel (17) aufweist, durch welches nach dem Verschließen eine haptische und/oder visuelle Rückmeldung für einen Anwender gegeben ist, so dass der Anwender ein ordnungsgemäßes Verschließen erkennt.

8. Probenbehälter (1) nach einem der vorstehenden Ansprüche, **dadurch gekennzeichnet, dass** die in Gebrauchsstellung durch Flüssigkeit verbundenen Kompartimente (6) durch von einem Boden (19) des Probenaufnahmeraums (2) abstehende Kompartimentwände (18) ausgebildet sind, insbesondere wobei die Kompartimentwände (18) gemessen vom Boden (19) eine geringere Höhe als wenigstens eine den Probenaufnahmeraum (2) begrenzende Wandung (7) haben.

9. Probenbehälter (1) nach einem der vorstehenden Ansprüche, **dadurch gekennzeichnet, dass** die Kompartimentwände (18) beabstandet zueinander angeordnet sind und/oder sich gegenseitig nicht kontaktieren.

10. Probenbehälter (1) nach einem der vorstehenden Ansprüche, **dadurch gekennzeichnet, dass** wenigstens ein Kompartiment (6), insbesondere jedes Kompartiment (6), eine zugeordnete Kupplungsstelle (8) und/oder eine Sollbruchstelle (11) aufweist.

11. Probenbehälter (1) nach einem der vorstehenden Ansprüche, **dadurch gekennzeichnet, dass** die wenigstens eine Sollbruchstelle (11) in radialer und/oder in axialer Richtung orientiert ist und/oder dass die wenigstens eine Sollbruchstelle (11) bei einer Kopplung des Probenbehälters (1) mit einem Zusatzbehälter (9) in radialer und/oder in axialer Richtung durchbrochen wird.

12. Probenbehälter (1) nach einem der vorstehenden Ansprüche, **dadurch gekennzeichnet, dass** die wenigstens eine sich bei erhöhenden Innendruck selbstverstärkende Dichtung (4, 5, 15, 16) durch einen sich in Richtung einer Behälteröffnung (20) verjüngenden Außenwandungsbereich (21) des Probenaufnahmeraums (2) ausgebildet ist, insbesondere wobei sich der Außenwandungsbereich in Schließstellung verformt.

13. Probenbehälter (1) nach einem der vorstehenden Ansprüche, **dadurch gekennzeichnet, dass** die wenigstens eine sich bei erhöhendem Innendruck selbstverstärkende Dichtung (4, 5, 15, 16) durch eine an einem Außenumfang einer oder der Außenwandung (7) des Probenaufnahmeraums (2) ausgebildete, insbesondere umlaufende, Materialausnehmung (22) und einer Seitenwand (14) eines oder des Deckels (13) ausgebildet ist, vorzugsweise wobei ein Innenumfang der Seitenwand über wenigstens einen Kontaktpunkt (23), insbesondere einen Steg, am Außenumfang der Außenwandung (7) abgestützt ist.

14. Probenbehälter (1) nach einem der vorstehenden Ansprüche, **dadurch gekennzeichnet, dass** die wenigstens eine sich bei erhöhendem Innendruck selbstverstärkende Dichtung (4, 5, 15, 16) durch einen Ringschnappverbinder (24) ausgebildet ist.

15. Verwendung eines Probenbehälters (1) nach einem der vorstehenden Ansprüche zur Kultivierung und/oder zur Analyse, insbesondere zur chemischen Analyse, einer Probe, **dadurch gekennzeichnet, dass** eine Flüssigkeit, insbesondere ein Nährmedium, im Probenaufnahmeraum (2) eingefüllt wird, und dass eine Probe in wenigstens einem Kompartiment (6) eines Probenaufnahmeraums (2) angeordnet wird.

16. Verwendung nach Anspruch 15, **dadurch gekennzeichnet, dass** eine Erwärmung der Flüssigkeit und/oder der Probe in einem der Kompartimente (6) vorgenommen wird, insbesondere wobei eine Temperatur der Flüssigkeit und/oder der Proben in wenigstens einem anderen Kompartiment (6) nahezu unverändert oder unverändert bleibt.

17. Verwendung nach einem der vorstehenden Ansprüche, **dadurch gekennzeichnet, dass** eine Flüssigkeit, insbesondere ein Nährmedium, im Probenaufnahmeraum (2) eingefüllt wird, so dass ein oberer Rand (25) der Kompartimentwände (18) der Kompartimente (6) jeweils unterhalb eines Flüssigkeitspegels (26) liegt.

18. Verwendung nach einem der vorstehenden Ansprüche, **dadurch gekennzeichnet, dass** durch die Flüssigkeit, insbesondere die die Kompartimentwände (18) überflutende Flüssigkeit, eine Verteilung, insbesondere eine gleichmäßige Verteilung, der Probe von einem Kompartiment (6) in wenigstens ein weiteres Kompartiment (6) vorgenommen wird, insbesondere indem der Probenbehälter (1) geschüttelt und/oder geschwenkt wird.
